# EUROPÄISCHE PATENTANMELDUNG

(11) **EP 2 353 600 A1**
(43) Veröffentlichungstag der Anmeldung: **10.08.2011**
(21) Anmeldenummer: 10004161.5
(22) Anmeldetag: 20.04.2010
(51) Int. Cl.: A61K 35/74, A61K 38/47

(54) **Mittel zur Anwendung bei Lactasemangel und Lactoseintoleranz**

(30) Priorität: 05.02.2010 DE 102010007142; 08.02.2010 DE 102010007304; 08.02.2010 DE 102010007289; 15.02.2010 DE 102010008083; 26.02.2010 DE 102010009582
(71) Anmelder: Vitacare GmbH & Co. KG, 60318 Frankfurt (DE)
(72) Erfinder: Wyrobnik, Daniel, 60322 Frankfurt (DE); Wyrobnik, Harry, 60431 Frankfurt (DE)

(57) **Zusammenfassung**

Es wird ein Mittel beschrieben, das mit Hilfe von Lactase und Milchsäurebakterien Lactose in Glucose und Galactose überführt und so die Bioverfügbarkeit von Lactose im menschlichen oder tierischen Organismus reduziert. Dieses Mittel kann insbesondere bei Lactoseintoleranz eingesetzt werden.

## Beschreibung

Gegenstand der Erfindung ist ein Mittel zur Anwendung bei Lactoseintoleranz, welches das Enzym Lactase (syn. Tilactase, Tilactasum, Beta-Galactosidase) in Kombination mit Mikroorganismen enthält. Dabei wird im Sinne dieser Patentanmeldung unter Lactoseintoleranz nicht nur die medizinisch definierte Lactoseintoleranz und Lactosestoffwechselstörung (Lactosemalabsorption, Lactosemaldigestion), sondern jede Form von gesundheitlichen Beeinträchtigungen und Beschwerden verstanden, die infolge der Zufuhr von Lactose oder lactosehaltiger Lebensmittel oder durch Freisetzung von Lactose im Verdauungstrakt eines Menschen oder Tieres aus anderen Stoffe wie z.B. Arzneimittel entstehen.

Lactose ist bekanntlich ein Zweifachzucker und stellt einen wichtigen Energie liefernden Nahrungsbestandteil dar. Sie kommt als Bestandteil vornehmlich in Milch und Milchprodukten vor, wird aber von der Lebensmittelindustrie auch als Süssstoff, Füllstoff und Konsistenzverbesserer eingesetzt, so z.B. in Süsswaren, Wurstwaren, Backwaren und Fertiggerichten. Auch Arzneimittel enthalten häufig Lactose. Im Organismus wird Lactose im Dünndarm durch das körpereigene Enzym Lactase in die verwertbaren Nährstoffe Glucose und Galactose gespalten. Für Babies ist die in der Muttermilch enthaltene Lactose ein sehr wichtiger Nährstoff.

Lactoseintoleranz ist in der Regel Folge eines Lactasemangels. Wenn das Enzym Lactase im Körper nicht ausreichend verfügbar ist (Lactasemangel), gelangt der Milchzucker in ungespaltener Form in die unteren Darmabschnitte (Dickdarm) und wird dort durch Darmbakterien unter Gasbildung vergoren. Dies kann zu Befindlichkeitsstörungen, wie z. B. Bauchschmerzen, Blähungen, Völlegefühl oder Durchfall, nach dem Verzehr von Milchprodukten führen. Außerdem kann die Lactose einen vermehrten Wassereinstrom in den Dickdarm verursachen. Man spricht dann von Lactose-Unverträglichkeit (Milchzucker-Unverträglichkeit) oder Lactoseintoleranz (Milchzuckerintoleranz). Wegen der Åhnlichkeit der Beschwerden wird Lactoseintoleranz häufig mit dem Reizdarmsyndrom (irritables Colon, colon irritabile) verwechselt.

Üblicherweise werden drei verschiedene Formen des Lactasemangels unterschieden:
1. Der primäre Lactasemangel
2. Der sekundäre Lactasemangel
3. Der angeborene Lactasemangel

Die Menge an Lactase im Dünndarm ist bei Babys während der Stillperiode am höchsten und nimmt dann bei den meisten Menschen genetisch bedingt kontinuierlich ab. Der sich dann ergebende sogenannte primäre Lactasemangel ist also das Resultat eines normalen Alterungsprozesses und bei der überwiegenden Mehrheit (70 % - 90 %) der erwachsenen Weltbevölkerung zu beobachten. So vertragen beispielsweise fast alle Bevölkerungsgruppen Afrikas und Asiens keinen Milchzucker. Aber auch in Deutschland haben ca. 15 % der Erwachsenen einen primären Lactasemangel. Die auf einem primären Lactasemangel beruhende Lactoseintoleranz wird dann häufig als primäre Lactoseintoleranz bezeichnet.

Verschiedene Darmerkrankungen können zu einem sogenannten sekundären Lactasemangel (syn. erworbener Lactasemangel) führen, so z. B. Morbus Crohn, Colitis ulcerosa, Zöliakie (einheimische Sprue) und sonstige Darmentzündungen (z. B. aufgrund von viralen oder bakteriellen Infekten). Auch nach Operationen im Magen- Darmbereich, besonders nach dem Entfernen von Teilen des Dünndarms, kommt es nicht selten zu einem sekundärem Lactasemangel. Man spricht dann häufig von der sich dann ergebenden sekundären oder transitorischen Lactoseintoleranz.
Der sekundäre Lactasemangel bildet sich nach der Ausheilung der ihn verursachenden Darmerkrankung üblicherweise wieder zurück.

Beim sehr selten vorkommenden angeborenen Lactasemangel (syn. kongenitaler Lactasemangel) fehlt den Neugeborenen das für die Lactaseproduktion verantwortliche Gen. Dies führt zu einer Unfähigkeit des Organismus das Enzym überhaupt zu bilden. Bei diesen Säuglingen muss eine strikt lactosefreie Ernährung eingehalten werden.

Im Sinne dieser Patentanmeldung wird unter Lactasemangel nicht nur der medizinisch definierte Lactasemangel verstanden, sondern jede Form eines Lactasemangels und jede Form von gesundheitlichen Beeinträchtigungen und Beschwerden, die infolge eines Lactasemangels nach der Zufuhr von Lactose oder lactosehaltigen Lebensmittel oder durch Freisetzung von Lactose im Verdauungstrakt eines Menschen oder Tieres aus anderen Stoffe wie z.B. Arzneimittel entstehen.

Nicht jede Störung des Lactose-Stoffwechsels führt notwendigerweise gleich zu einer schweren Lactoseintoleranz. Jedoch sind auch bei leichten Störungen des Lactose-Stoffwechsels Befindlichkeitasstörungen zu beobachten.
Die oben genannten Befindlichkeitsstörungen konnten bisher nur durch das Einhalten einer lactosearmen oder lactosefreien Diät oder durch den mit lactosehaltigen Speisen gleichzeitigen Verzehr von Präparaten, die das Enzym Lactase enthalten, vermieden werden Das Einhalten einer lactosearmen oder lactosefreien Diät ist unbefriedigend und wegen den in Milchprodukten enthaltenen wertvollen Nähr- und Vitalstoffen auch ernährungsphysiologisch ungünstig. Die Auswahl an lactosefreien Milchprodukten ist beschränkt und ausserdem schmecken diese wegen des bereits bei der Produktion durch den Zusatz von Lactase gespaltenen Milchzuckers süsser als lactosehaltige Milchprodukte. Vielen Verbrauchern schmecken diese Produkte nicht. Hinzu kommt, dass sehr viele Lebensmittel, von denen man es nicht erwarten würde, Milchzucker enthalten. Daher macht der Einkauf milchzuckerfreier Lebensmittel häufig Schwierigkeiten. Die zeitnahe Verwendung von Lactasepräparaten stellt eine gute Alternative dar und erhöht die Lebensqualität der Betroffenen häufig erheblich. Die Menge an individuell benötigter Lactase hängt einerseits von der Empfindlichkeit des Verwenders gegenüber der Lactose ab. Diese ergibt sich im Wesentlichen aus der Restaktivität der körpereigenen Lactase. Außerdem hängt die benötigte Lactasemenge von der Art der Nahrung, der Gesamtmenge der Nahrung und der in ihr enthaltenen Lactosemenge ab. Es ist bekannt, dass Lactasepräparate nicht bei allen Betroffenen und immer gleich gut wirken. Ein Mittel, welches eine zuverlässigere und/oder bessere Wirkung als nur das Enzym Lactase enthaltene Präparate hätte, würde also für die vielen Betroffenen ein bestehendes dringendes Bedürfniss befriedigen und eine erhebliche Verbesserung und sprunghafte Weiterentwicklung der Ernährungsmöglichkeiten bei Lactoseintoleranz bedeuten.

Somit ist es die Aufgabe der vorliegenden Erfindung ein wirksames Mittel zur Anwendung bei Lactoseintoleranz zur Verfügung zu stellen, um die Einnahme lactosehaltiger Lebensmittel auch bei Lactoseintoleranz zu ermöglichen. Ferner ist es eine Aufgabe der Erfindung Lactoseintoleranz-Betroffenen Lebensmittel zugänglich zu machen, die ihnen bisher aufgrund ihres Lactosegehaltes zu verzehren verwehrt waren. Des Weiteren soll ein Mittel bereit gestellt werden, welches nach Einnahme von lactosehaltigen Lebensmittel oder lactosehaltigen Stoffen das Auftreten von Lactoseseintoleranz-Symptomen, verhindern, verringern oder beseitigen kann. Die mit den Ansprüchen 1 bis 16 beschriebene Lösung dieser Aufgaben ist Teil der Beschreibung.

Gegenstand der Erfindung ist deshalb ein Mittel, welches die vorstehend beschriebenen Probleme lösen kann. Das Mittel enthält das Enzym Lactase in Kombination mit Mikroorganismen. Bei den Mikroorganismen handelt es sich um Milchsäurebakterien und besonders bevorzugt um Lactobacillus delbrueckii subspecies bulgaricus und/oder Streptococcus thermophilus. Eine Lactase im Sinne dieser Erfindung ist ein Enzym, das in der Lage ist, Lactose in Glucose und Galactose zu überführen (aufzuspalten). Milchsäurebakterien im Sinne dieser Erfindung sind insbesondere Mikroorganismen, die mittels ihrer eigenen mikrobiellen Lactase Lactose verstoffwechseln können und/oder aus denen im Verdauungstrakt eines Menschen oder Tieres Lactase freigesetzt werden kann. Die erfindungsgemäß zum Einsatz kommenden Mikroorganismen können vorzugsweise gefriergetrocknet sein, um eine längere Haltbarkeit und gute Verarbeitbarkeit zu gewährleisten.

Wenn im folgenden von Lactobacillus bulgaricus die Rede ist, so ist Lactobacillus delbrueckii subspecies (subsp.) bulgaricus gemeint.

Das erfindungsgemäße Mittel kann bewirken, das Lactose in der Nahrung bzw. im Nahrungsbrei nach dem Verzehr in Glucose und Galactose gespalten wird. Dadurch steht die Lactose für den durch Vergärung gekennzeichneten bakteriellen Stoffwechsel im Darm nicht mehr zur Verfügung.

Gegenstand der Erfindung ist deshalb ein Mittel, welches mit Hilfe von Lactase in Kombination mit Mikroorganismen, vorzugsweise Milchsäurebakterien, besonders bevorzugt Lactobacillus bulgaricus und/oder Streptococcus thermophilus, die Bioverfügbarkeit von Lactose im menschlichen oder tierischen Organismus reduziert.

Gegenstand der Erfindung ist auch ein Mittel, welches mit Hilfe von Lactase in Kombination mit Mikroorganismen, vorzugsweise Milchsäurebakterien, besonders bevorzugt Lactobacillus bulgaricus und/oder Streptococcus thermophilus, die für den menschlichen oder tierischen Organismus oder für in selbigen siedelnden Darmbakterien zur Verfügung stehenden Lactosemenge vermindert.

Gegenstand der Erfindung ist ferner ein Mittel zur Anwendung bei Lactoseintoleranz, das Lactase in Kombination mit Mikroorganismen, vorzugsweise Milchsäurebakterien, besonders bevorzugt Lactobacillus bulgaricus und/oder Streptococcus thermophilus, enthält.

Ein weiterer Gegenstand der Erfindung ist die Verwendung von Lactase in Kombination mit Mikroorganismen, vorzugsweise Milchsäurebakterien, besonders bevorzugt Lactobacillus bulgaricus und/oder Streptococcus thermophilus, bei Lactoseintoleranz.

Die Lactase ist ein Enzym, das die Eigenschaft besitzt, Lactose In Glucose und Galactose zu überführen. Während Lactose nicht aus dem Dünndarm resorbiert wird, handelt es sich bei der Glucose und der Galactose um Einfachzucker, die leicht verdaulich sind und schnell resorbiert werden. Die heutzutage in zur oralen Verwendung bestimmten Lactasepräparaten üblicherweise verwendeten Lactasen sind sogenannte saure Lactasen, weiche meist mittels Aspergillus oryzae hergestellt werden. Saure Lactase wirkt gut im sauren Mileu des nach einer Nahrungsaufnahme befüllten Magens. Saure Lactase ist stabil und wirksam bei einem pH von ca. 3 - 6. Im Sinne dieser Anmeldung bedeutet der Begriff "stabil", dass die saure Lactase bei 37 °C und pH-Werten von 3 - 8 nach einer Stunde noch mindestens 30 % ihrer Aktivität besitzt. Bei einem pH-Wert von über 6,5, wie er im Dünndarm vorherrscht, ist saure Lactase zwar stabil aber nur noch wenig aktiv. Im Dünndarm kann die saure Lactase daher keine befriedigende Wirkung mehr entfalten. Bisherige Lactasepräparate müssen daher möglichst so viel Lactase enthalten, dass üblicherweise mit Lebensmitteln verzehrte Mengen an Lactose möglichst weitgehend bereits im Magen gespalten werden. Eine Lactase, die auch bei niedrigen pH-Werten stabil und aktiv ist und sowohl gut im sauren Mileu des Magens als auch gut im Dünndarm wirken kann ist nicht bekannt und kommerziell nicht erhältlich. Da in Abhängigkeit der verzehrten Nahrung und der verzehrten Nahrungsmenge die Passagezeit im Magen stark variiert, stellt die richtige Dosierung von Lactasepräparaten für die Anwender häufig ein Problem dar und die Folge ist, wie bereits geschildert, dass Anwender mit der Wirkung dieser Produkte nicht selten unzufrieden sind. Eine Erhöhung der Lactaseaktivität pro Dosiseinheit kann zwar hilfreich sein, jedoch hat sich gezeigt, dass die Wirkungszunahme und Wirkzuverlässigkeit nicht proportional zur Lactaseaktivität je Dosiseinheit steigt. Auch der Zeitpunkt der Einnahme ist kritisch, da die saure Lactase im unbefüllten Magen bei dem dann vorherrschenden sehr niedrigen pH-Wert (zwischen 1 und 2,5) schnell inaktiviert wird. Wenn ein Verbraucher ein Lactasepräparat also zu lange (z.B. 10 Minuten vorher) vor einer Mahlzeit auf nüchternen Magen verzehrt, so kann es sein, dass das Enzym zum Zeitpunkt der Nahrungsaufnahme bereits seine Aktivität irreversibel verloren hat und dann für die gewünschte Lactosespaltung nicht mehr zur Verfügung steht, so dass es in diesem Fall auch nicht auf die Lactaseaktivität je Dosiseinheit ankommt. Bestimmte Milchsäurebakterien enthalten eine Lactase, die bei den im Dünndarm vorherrschenden pH-Werten aktiv und stabil ist. Die Milchsäurebakterien-Lactase kann durch die Bakterienhülle geschützt in den Dünndarm gelangen und dort können dann zwei vorteilhafte Effekte zum Tragen kommen. Zum einen kann die mikrobielle Lactase freigesetzt werden, wenn die Bakterienhülle im Dünndarm durch die Verdauungssäfte zerstört wird, so dass Lactose, die im Magen durch die saure Lactase noch nicht gespalten wurde, dann von dieser von den Milchsäurebakterien freigesetzten Lactase gespalten wird. Zum anderen können intakt gebliebene Bakterien Lactose zur eigenen Energiegewinnung verstoffwachseln und so ebenfalls zu einer weiteren Reduzierung der Lactosemenge im Dünndarm beitragen. Auf dem Lactasegehalt von Milchsäurebakterien beruht teilweise die bei Lactoseintoleranz bei einigen Betroffenen manchmal bessere Verträglichkeit von fermentierte Milchprodukten gegenüber unfermentierten Milchprodukten. Die meisten Betroffenen können jedoch auch fermentierte Milchprodukte nicht problemlos verzehren. In Studien, bei denen Lactoseintoleranten Milchsäurebakterien gegeben wurden, hat sich gezeigt, dass diese keine gute und zuverlässige Wirksamkeit in Bezug auf die Lactoseverdauung haben. Diese Ergebnisse haben sich für eine Vielzahl von verschiedenen Stämmen gezeigt, auch wenn diese in den verschiedensten Kombinationen verwendet wurden. Die Verwendung eines Präparates bei Lactoseintoleranz, welches ausschließlich Milchsäurebakterien enthält, ist daher nicht sinnvoll. Während heutzutage häufig versucht wird, Milchprodukten und anderen Nahrungsmitteln solche Milchsäurebakterien zuzusetzen, die intakt den Dickdarm erreichen, um dort für die Gesundheit vorteilhafte Effekte auszuüben, wird bei der hier vorgestellten Erfindung gewünscht, dass möglichst viele der eingesetzten Milchsäurebakterien im Dünndarm zerstört werden, damit dort deren mikrobielle Lactase freigesetzt wird, welche dann für die Lactosespaltung zur Verfügung steht. Durch den Zusatz solcher Milchsäurebakterien kann die Wirksamkeit und/oder Zuverlässigkeit von Lactasepräparaten erhöht werden. Ein solches Präparat ist einerseits in der Lage Lactose im Magen (durch die saure Lactase) zu spalten und andererseits in der Lage die Lactose, die im Magen nicht gespalten wurde im Dünndarm (mittels der Milchsäurebakterien-Lactase) zu spalten. Es wird also durch den Zusatz der Milchsäurebakterien eine Wirkverlängerung des Präparates erreicht, welche zu einer höheren Wirksamkeit und/oder Wirkzuverlässigkeit führt, die durch eine reine Erhöhung der Lactaseaktivität je Dosiseinheit nicht oder zumindest nicht ebenso kosteneffektiv erreicht werden kann. Es ist also vorteilhaft dem erfindungsgemäßen Mittel zusätzlich zu dem Enzym Lactase solche Mikroorganismen hinzuzufügen, die Ihre eigene im Dünndarm ausreichend aktive und stabile Lactase freisetzen und/oder Lactose im Dünndarm verstoffwechseln. Es wurde gefunden, dass die Milchsäurebakterien Lactobacillus bulgaricus und Streptococcus thermophilus für diese Verwendung besonders geeignet sind, da diese Mikroorganismen überwiegend, zumindest jedoch teilweise, mit intakter die mikrobielle Lactase schützender Bakterienhülle den Magen überstehen, aber den Dünndarm überwiegend, zumindest jedoch teilweise, nicht intakt überstehen und somit ihre mikrobielle Lactase dort in relevantem Umfang freisetzen. Ausserdem kommen beispielsweise in Frage: Lactobacillus delbrueckii (andere subsp. als Lactobacillus bulgaricus), Lactobacillus acidophilus, Lactobacillus rhamnosus, Lactobacillus casei, Lactobacillus paracasei, Lactobacillus brevis, Lactobacillus planterum, Lactobacillus salivarius, Lactobacillus rheuteri, Lactococcus lactis, Pediococcos acidilacti, Bifidobacterium lactis, Bifidobacterium bifidum, Bifidobacterium breve, Bifidobacterium longum, Bifidobacterium infantis.

Ein derartiges Kombinationsmittel, kann auch in Form von zwei getrennten Dosiseinheiten, z.B. in zwei getrennten Tabletten oder Kapseln, eingesetzt werden, von denen die eine das Enzym Lactase und die andere die Milchsäurebakterien enthält.

Lebensmitteln im Sinne dieser Erfindung sind Lebensmittel im Sinne der Verordnung (EG) Nr. 178/2002 des Europäischen Parlaments und des Rates vom 28.01.2002. Zu den Lebensmittel im Sinne dieser Erfindung zählen insbesondere auch diätetische Lebensmittel, diätetische Lebensmittel für besondere medizinische Zwecke, Nahrungsergänzungsmittel und Lebensmittelzusätze.

Nahrungsergänzungsmittel im Sinne dieser Erfindung sind Nahrungsergänzungsmittel im Sinne der Richtlinie 2002/46/EG des europäischen Parlaments und des Rates vom 10. Juni 2002.
Diätetische Lebensmittel im Sinne dieser Erfindung sind diätetische Lebensmittel im Sinne der Richtlinie 2009/39/EG des europäischen Parlaments und des Rates vom 6. Mai 2009 über Lebensmittel die für eine besondere Ernährung bestimmt sind.
Diätetische Lebensmittel für besondere medizinische Zwecke im Sinne dieser Erfindung sind Diätetische Lebensmittel für besondere medizinische Zwecke im Sinne der Richtlinie 1999/21/EG der Kommission vom 25. März 1999 über diätetische Lebensmittel für besondere medizinische Zwecke

Erfindungsgemäß wird das Enzym Lactase in Kombination mit Milchsäurebakterien, insbesondere in Kombination mit Lactobacillus bulgaricus und/oder Streptococcus thermophilus zur Verwendung in der Medizin, beispielsweise als Arzneimittel, angegeben. Gegenstand der Erfindung ist dementsprechend auch ein Erzeugnis, das aus Lactase in Kombination mit Lactobacillus bulgaricus und/oder Streptococcus thermophilus besteht oder diese Stoffe neben einem oder mehreren anderen Wirkstoffen enthält, zur Anwendung in einem medizinischen Verfahren, insbesondere einem Verfahren zur therapeutischen Behandlung des menschlichen oder tierischen Körpers. Gegenstand der Erfindung ist auch eine Zusammensetzung zur Verwendung in der Medizin, enthaltend eine Lactase und einen oder mehrere Lactase-bildende Mikroorganismen, ausgewählt aus Lactobacillus bulgaricus, Lactobacillus delbrueckii, Lactobacillus acidophilus, Lactobacillus rhamnosus, Lactobacillus casei, Lactobacillus paracasei, Lactobacillus brevis, Lactobacillus plantarum, Lactobacillus salivarius, Lactobacillus rheuteri, Lactobacillus lactis, Pediococcus acidilacti, Bifidobacterium lactis, Bifidobacterium bifidum, Bifidobacterium breve, Bifidobacterium longum, Bifidobacterium infantis und Steptococcus thermophilus, wobei die Lactase vorzugsweise nicht aus dem bzw. den vorgenannten Mikroorganismen stammt.
Im Sinne dieser Erfindung ist ein Arzneimittel ein Erzeugnis, im Sinne der Richtlinie 2001/83 EG in der Fassung der Richtlinie 2004/27 EG.

Gemäß einem weiteren Aspekt wird erfindungsgemäß ein Lebensmittel bereitgestellt, das Lactase in Kombination mit Milchsäurebakterien, insbesondere in Kombination mit Lactobacillus bulgaricus und/oder Streptococcus thermophilus, enthält. Ferner wird erfindungsgemäß ein Lebensmittel bereitgestellt, dass Lactase in Kombination mit Milchsäurebakterien, insbesondere in Kombination mit Lactobacillus bulgaricus und/oder Streptococcus thermophilus, in einer zum Überführen von Lactose zu Glucose und Galactose wirksamen Menge enthält.

Ein Lebensmittel kann auch durch ein Verfahren hergestellt werden, bei dem das Lebensmittel mit Lactase in Kombination mit Lactobacillus bulgaricus und/oder Streptococcus thermophilus - auf eine Weise versetzt wird, bei welcher die Wirkung der Lactase und der Milchsäurebakterien überwiegend nach dem Verzehr des Lebensmittels einsetzt. Ein solches Lebensmittel besitzt einen weitgehend gleichen Geschmack wie ein unbehandeltes Lebensmittel und ist aufgrund des sich nach dem Verzehr einstellenden reduzierten Lactosegehalts zum Verzehr bei Lactoseintoleranz geeignet. Überwiegend im Sinne dieser Anmeldung bedeutet, dass die milchzuckerspaltende Wirkung des Enzyms und der Milchsäurebakterien zu mindestens 50 % nach dem Verzehr des Lebensmittels einsetzt. Alternativ kann die Wirkung (a) zu mindestens 90 %, (b) zu mindestens 80 %, (c) zu mindestens 70 % oder (d) zu mindestens 60 % erst nach dem Verzehr des Lebensmittels einsetzen.

Gemäß einem weiteren Aspekt wird erfindungsgemäß ein Medizinprodukt bereitgestellt, welches Lactase in Kombination mit Milchsäurebakterien, insbesondere in Kombination mit Lactobacillus bulgaricus und/oder Streptococcus thermophilus, enthält. Gegenstand der Erfindung ist dementsprechend auch ein Medizinprodukt, das aus Lactase in Kombination mit Milchsäurebakterien, insbesondere in Kombination mit Lactobacillus bulgaricus und/oder Streptococcus thermophilus, besteht oder diese neben einem oder mehreren anderen Wirkstoffen enthält.

Ein Medizinprodukt im Sinne dieser Patentanmeldung sind Medizinprodukte im Sinne der Richtlinie 93/42 EWG in der durch die Richtlinie 2007/47/EG geänderten Fassung.

Die Erfindung wird nachfolgend in ihren verschiedenen Aspekten weiter beschrieben. Soweit nachfolgend von einem Mittel gesprochen wird, ist dabei immer auch ein Lebensmittel, Medizinprodukt oder Arzneimittel gemeint.

Lactase wurde bisher noch nicht in Kombination mit Lactobacillus bulgaricus und/oder Streptococcus thermophilus im medizinisch-pharmazeutischen Bereich, insbesondere bei Lactoseintoleranz bei Menschen oder Tieren, angewendet. Folglich handelt es sich bei der hier vorgestellten Erfindung um die erste medizinische Indikation für die Kombination aus diesen Stoffen.

Die erfindungsgemäßen Mittel können peroral vor den Mahlzeiten, zu den Mahlzeiten oder unmittelbar danach eingenommen werden, damit sie im Nahrungsbrei ihre spaltende Wirkung auf die Lactose entfalten können. Vorzugsweise werden die erfindungsgemäßen Mittel unmittelbar vor den Mahlzeiten während den Mahlzeiten oder unmittelbar nach den Mahlzeiten eingenommen. Die erfindungsgemäßen Mittel können das Enzym und die Milchsäurebakterien ohne weitere Zusatzstoffe enthalten. Es ist jedoch bevorzugt, daß die erfindungsgemäßen Mittel weiterhin pharmazeutisch verträgliche und/oder lebensmittelunbedenkliche Zusatzstoffe enthalten, wie beispielsweise Streckmittel, Bindemittel, Stabilisatoren, Konservierungsmittel, Geschmacksstoffe etc. Solche Zusatzstoffe sind für die Zubereitung von Arzneimitteln, Medizinprodukten, Lebensmitteln, diätetischen Lebensmitteln, diätetischen Lebensmittel für besondere medizinische Zwecke, Nahrungsergänzungsmitteln und Lebensmittelzusätzen üblich und gut bekannt, und der Fachmann auf dem Gebiet weiß, welche Zusatzstoffe in welchen Mengen für bestimmte Darreichungsformen geeignet sind. Besonders bevorzugt enthalten die erfindungsgemäßen Mittel als Zusatzstoffe Di-Calcium-Phosphat, Lactose, modifizierte Stärke, mikrokristalline Cellulose, Maltodextrin und/oder Fibersol.

Die erfindungsgemäßen Mittel können einem Lebensmittel auch vor dem Verzehr zugesetzt werden. Sie können dem Lebensmittel sogar schon bei der Herstellung mit dem Ziel zugesetzt werden, ihre Wirkung überwiegend erst nach dem Verzehr des Lebensmittels zu entfalten. Dies könnte gegebenenfalls z.B. durch eine Mikroverkapselung erreicht werden. Dadurch würde der verwertbare Lactosegehalt des Lebensmittels auf besonders vorteilhafte Weise ohne Nachteile für dessen Geschmack reduziert werden. Bevorzugt sind deshalb Zubereitungen enthaltend Lactase in Kombination mit Lactobacillus bulgaricus und/oder Streptococcus thermophilus, die diese Stoffe überwiegend erst im Verdauungstrakt eines Menschen oder Tieres freisetzen oder anderweitig wirksam werden lassen, insbesondere im Magen oder Dünndarm. Die Erfindung könnte daher zum Beispiel bei der Herstellung von Milch und Milchprodukten, wie z.B. Quark, Joghurt, Sahne, Käse, Pudding, Milchgetränke, Milchmixgetränke Elskrem, und bei der Herstellung von z.B. Schokolade und Schokoladenerzeugnissen, Backwaren (z.B. Kekse und Kuchen), Brotwaren, lactosehaltigen Getränken, lactosehaltigen Saucen (z.B. Sahnesaucen) und lactosehaltigen Süßstoffen verwendet werden. Bei Speisen, die gekocht oder gebacken werden, könnten die erfindungsgemäßen Mittel nach dem Abkühlen z.B. untergemischt oder aufgestreut werden. Sofern es sich um Milch und Milchprodukte handelt, so enthalten diese erfindungsgemäß saure Lactase und mindestens einen der in dieser Anmeldung aufgeführten Mikroorganismen, vorzugsweise Lactobacillus bulgaricus und Streptoccocus thermophilus, jedoch nicht Lactobacillus acidophilus.

Die erfindungsgemäßen Mittel können auch einem Lebensmittel zugesetzt werden, um ihre Wirkung nach dem Verzehr an der aus einem anderen Lebensmittel stammenden Lactose zu entfalten. Ein Beispiel hierfür wäre der Zusatz der erfindungsgemäßen Mittel zu Cerealien, so daß die Reduzierung der in der Milch enthaltenen Lactose nach dem Verzehr ders mit der Milch zubereiteten Cerealien eintritt, ohne den Geschmack der Milch zu beeinträchtigen.

Erfindungsgegenstand sind auch Mittel, die neben anderen Wirkstoffen zusätzlich auch noch Lactase in Kombination mit Lactobacillus bulgaricus und/oder Streptococcus thermophilus enthalten.

Die Erfindung kann in jeder für den beabsichtigten Verabreichungsweg geeigneten Form formuliert sein. Für die orale Verabreichung sind die erfindungsgemäßen Mittel vorzugsweise in der Form von Kapseln (überzogen oder nicht überzogen), Tabletten (überzogen oder nicht überzogen), Dragees (überzogen oder nicht überzogen), Kapseln, die überzogene oder nicht überzogene Pellets, Granulat oder Mikro bzw. Minitabletten enthalten, Tabletten, die aus überzogenen oder nicht überzogenen Pellets, oder Mikro- bzw. Minitabletten oder überzogenem oder nicht überzogenem Pulver gepreßt sind. Möglich für die orale Verabreichung sind auch überzogene oder nicht überzogene Gelkappen (Weichgelatinekapseln) oder flüssige Formen, wie z.B, eine Lösung, Tropfen, eine Suspension oder ein Gel. Die Erfindung kann auch als getrocknete orale Ergänzung oder feuchte orale Ergänzung vorliegen. Die Formulierung der erfindungsgemäßen Mittel als Pulver eignet sich besonders zur Beimischung zu einem Lebensmittel, Das Pulver kann auf eine Mahlzeit aufgestreut werden, oder es kann einem Brei oder einem Getränk untergemischt werden. Besonders zweckmäßig ist es, wenn das als loses Pulver angebotene Mittel in einzelnen Dosierungsmangen abgepackt wird, wie beispielsweise in einzelnen Beuteln oder wenn es in einem Dosierspender bereitgestellt wird. Ganz besonders bevorzugt werden die erfindungsgemäßen Mittel als Pulver oder Granulat oder Pellets in Kapseln oder als Tablette formuliert, die oral verabreicht werden.

Zur oralen Verabreichung können die Wirkstoffe in verträglichen Exzipienten und/oder Trägern enthalten sein. Der Ausdruck "verträglicher Träger" bezieht sich auf einen Träger, welcher den Wirkstoff zu seiner Wirkungsstelle liefert und dem Empfänger Mensch oder Tier keinen erheblichen Schaden zufügen wird. Die konkrete Form des Trägers ist jedoch nicht entscheidend.

Die Gesamtmenge des Trägers und/oder Exzipienten an einem erfindungsgemäßen Mittel beträgt bevorzugt zwischen 5 und 99,9 Gew.-%, stärker bevorzugt zwischen 10 und 95 Gew.-% und noch stärker bevorzugt zwischen 25 und 90 Gew.-% der Zusammensetzung.

Geeignete Exziplenten und/oder Träger schließen Maltodextrin, Calciumcarbonat, Di-Calcium-Phosphat, Tricalciumphosphat, mikrokristalline Cellulose, Dextrose, Reismehl, Magnesiumstearat, Stearinsäure, Croscarmellose-Natrium, Natriumstärkeglycolat, Crospovidon, Saccharose, pflanzliche Gummis. Lactose, Methylcellulose, Povidon, Carboxymethylcellulose, Maisstärke, modifizierte Stärke, Fibersol, Gelatine, Hydroxypropylmethylcellulose und dergleichen (einschließlich Gemischen davon) ein. Bevorzugte Träger schließen Calciumcarbonat, Magnesiumstearat, Maltodextrin, Di-Calcium-Phosphat, modifizierte Stärke, mikrokristalline Cellulose, Fibersol, Gelatine, Hydroxypropylmethycellulose und Gemische davon ein. Die unterschiedlichen Bestandteile und der Exzipient und/oder Träger werden vermischt und unter Verwendung herkömmlicher Verfahren in die gewünschte Form geformt, Die erfindungsgemäße zur oralen Verabreichung bestimmte Darreichungsform, wie z.B. Tablette oder Kapsel, kann mit einem gegen niedrige pH-Werte resistenten Überzug oder mit einem magensaftresistentem Überzug überzogen sein. Es kann auch ein Überzug zur Anwendung kommen, welcher zwar nicht gegen niedrige pH-Werte resistent ist, jedoch die Freisetzung des jeweiligen Enzyms bzw. des jeweiligen Milchsäurebakteriums bei niedrigen pH-Werten verzögert. Es ist auch möglich das erfindungsgemäße Mittel als überzogene (siehe oben) Pellets, Granulat oder Mikro- bzw. Minitabletten herzustellen, welche in nicht überzogene Kapseln gefüllt oder in nicht überzogene Tabletten gepreßt werden können. Geeignete Überzüge sind beispielsweise Celluloseacetatphthalat, Cellulosederivate, Schellack, Polyvinylpyrrolidon-Derivate, Acrylsäure, Polyacrylsäurederivate und Polymethylmethacrylate (PMMA), wie z.B. Eudragit (der Röhm GmbH, Darmstadt), Insbesondere Eudragit FS30D und Eudragit L30D-55. Durch den Zusatz von z.B. Natronlauge zum Überzugsmittel Eudragit, kann die pH-Wert Resistenz dieses Überzugsmittels zusätzlich beeinflußt werden. Weitere Details über Verfahren der Formulierung und Verabreichung können in der 21. Auflage von "Remington: The Science & Practice of Pharmacy", erschienen 2005 im Verlag Lippincott, Williams & Wilkins, Baltimore, USA und in Prof. Bauer "Lehrbuch der Pharmazeutischen Technologie", 18. Auflage erschienen in 2006 im Verlag Wissenschaftliche Verlagsgesellschaft (ISBN 3804-72222-9) gefunden werden, wobei die Dokumente hiermit durch Bezugnahme aufgenommen werden.

Obwohl erfindungsgemäß vorzugsweise solche Milchsäurebakterien zum Einsatz kommen, die den Magen möglichst mit intakter Bakterienhülle überstehen, kann es dennoch vorteilhaft sein, die Milchsäurebakterien mit einem magensaftresistenten Überzug zu versehen oder in z.B. Kapseln, Dragees oder Tabletten zu formulieren, welche mit einem magensaftresistenten Überzug versehen werden, damit ein möglichst großer Teil der Bakterien mit intakter Bakterienhülle den bereits zum Dünndarm gehörenden Zwölffingerdarm erreicht. Auch könnten die Milchsäurebakterien in z.B. magensaftresistent beschichteten Pellets enthalten sein oder aber das Milchsäurepulver könnte mit einem magensaftresistenten Überzug versehen werden (z,B, mittels eines Sprühverfahrens). Die Pellets oder das Pulver könnten in unbeschichtete Kapseln gefüllt werden. Diese könnten auch die Lactase, welche ja im Magen freigesetzt werden soll enthalten. Eine solche, die Milchsäurebakterien vor der Magensäure schützende, Beschichtung wäre insbesondere dann sehr vorteilhaft, wenn Milchsäurebakterien zum Einsatz kommen, deren Bakterienhülle sonst im Magen zerstört würde, so dass deren mikrobielle Lactase dann im Magen wegen des dort niedrigen pH-Wertes inaktiviert würde.

Andere geeignete pharmazeutisch verträgliche Träger bzw. Hilfsstoffe zur Verwendung in der vorliegenden Erfindung schließen ein, sind aber nicht beschränkt auf, Wasser, Mineralöl, Ethylenglycol, Propylenglycol, Lanolin, Glycerylstearat, Sorbitanstearat, Isopropylmyristat, Isopropylpalmitat, Aceton, Glycerin, Phosphatidylcholin, Natriumcholat oder Ethanol.

Die Zusammensetzungen zur Verwendung in der vorliegenden Erfindung können außerdem mindestens einen Coemulgator umfassen, welcher einschließt, aber nicht beschränkt ist auf, oxyethyleniertes Sorbitanmonostearat, Fettalkohole, wie Stearylalkohol oder Cetylalkohol, oder Ester von Fettsäuren und Polyolen, wie Glycerylstearat.

Vorzugsweise werden die in der vorliegenden Erfindung zu verwendenden Mittel in einer stabilisierten Form zur Verfügung gestellt. Im Allgemeinen schließen Stabilisierungsmethoden und verfahren, die gemäß der vorliegenden Erfindung verwendet werden können, jegliche und alle auf dem Fachgebiet bekannten Verfahren zur Stabilisierung von chemischem oder biologischem Material ein, umfassend z. B. den Zusatz von chemischen Mitteln, Methoden auf Basis von Temperaturmodulation; Methoden auf Basis von Bestrahlung oder Kombinationen davon. Chemische Mittel, die gemäß der vorliegenden Erfindung verwendet werden können, schließen unter anderem Konservierungsmittel; Säuren, Basen; Salze; Antioxidanzien; Viskositätsverbesserer; Emulgatoren; Geliermittel; und Gemische davon ein.

Saure Lactase ist kommerziell erhältlich (z.B. Amano Japan und DSM Niederlande) und wird üblicherweise mit Hilfe des Mikroorganismus Aspergillus oryzae mikrobiologisch hergestellt. Lactobacillus bulgaricus und Streptococcus thermophilus sind ebenfalls kommerziell erhältlich (z.B. Danisco, Dänemark oder Christian Hansen, Dänemark), Die Erfindung ist jedoch nicht auf die derzeit kommerziell erhältlichen saure Lactase-Enzyme beschränkt, sondern betrifft ganz allgemein Enzyme, die die Umsetzung von Lacto8e , spezifisch oder unspezifisch - zu Glucose und Galactose bewirken können in Kombination mir Milchsäurebakterien, die im Dünndarm Lactose abbauen können, und zwar entweder durch Freisetzung von Lactase oder durch intrazelluläre Verstoffwechselung von Lactose.

Die Aktivität der Lactase wird erfindungsgemäß in FCC-Einheiten (Food Chemical Codex-Einheiten) definiert. Eine Enzymeinheit setzt bei 37°C und pH 4,5 ein Micromol o-nitrophenol pro Minute frei, und zwar unter den Bedingungen, wie Sie In der aktuellen sechsten Auflage des Food Chemical Codex beschrieben werden (siehe dort gesamte Messvorschrift, Testbeschreibung, Aktivltätsberechnung).

Bei einer nach dieser Definition bestimmten Enyzmaktivität sollte das erfindungsgemäße Mittel je Dosiseinheit 500 bis 30.000 FCC-Einheiten, vorzugsweise 2000 bis 20.000 FCC-Einheiten und besonders bevorzugt 3000 bis 10.000 FCC-Einheiten Lactase enthalten,

Wenn im folgenden Mengenangaben (in colony forming units, abgekürzt cfu) bezüglich der im erfindungsgemäßen Mittel enthaltenen Milchsäurebakterien gemacht werden, so beziehen sich diese auf die Menge an noch lebensfähigen Milchsäurebakterien (in cfu) am Ende der Haltbarkeit des erfindungsgemäßen Mittels. Es ist zu beachten, dass Milchsäuerbakterien enthaltende Präparate vorzugsweise trocken und kühl zu lagern sind. So kann es, um eine ausreichend lange lebensfähigkeit der Milchsäurebakterien zu gewährleisten vorteilhaft sein, das erfindungsgmäße Mittel in einem luftdichten Gefäß (z.B. aus Glas oder Aluminium) zu lagern, welches Ober ein Trockenmittel (z.B. im Verschluss), wie z.B. Silicagel oder Molokularsiebe, verfügt.

Die Menge an Milchsäurebakterien wird in cfu (colony forming units) angegeben. Das erfindungsgemäß Mittel sollte je Milchsäurebakterienstamm zwischen 10 Millionen (Mio.) und 200 Milliarden (Mrd.) cfu bevorzugt zwischen 50 Mio. und 10 Mrd. cfu und besonders bevorzugt zwischen 50 Mio. und 2 Mrd. cfu enthalten.

Im Falle eines Kombinationsmittels welches zusätzlich zum Enzym Lactase Lactobacillus bulgaricus und/oder Streptococcus thermophilus enthält sollte eine Dosiseinheit zwischen 10 Mio, und 200 Mrd. cfu Lactobacillus bulgaricus, bevorzugt zwischen 50 Mio. und 10 Mrd. cfu Lactobacillus bulgaricus und besonders bevorzugt zwischen 50 Mio und 2 Mrd. cfu Lactobacillus bulgaricus enthalten. Zusätzlich oder statt den Lactobacillus bulgaricus Bakterien sollte das erfindunsggemäße Mittel je Dosiseinheit zwischen 10 Mio. und 200 Mrd. cfu Streptococcus thermophilus, bevorzugt zwischen 50 Mio. und 10 Mrd. cfu Streptococcus thermophilus und besonders bevorzugt zwischen 50 Mio.und 2 Mrd, Streptococcus thermophilus enthalten.

Als besonders vorteilhaft hat sich ein erfindungsgemäßes Mittel herausgestellt, welches je Dosiseinheit zwischen 2000 und 6000 FCC-Einheiten saure Lactase, zwischen 100 Mio. und 2 Mrd. cfu Lactobacillus bulgaricus und zwischen 100 Mio und 2 Mrd. cfu Streptococcus thermophilus enthält. Besonders vorteilhafte Ergebnisse lassen sich erzielen, wenn die saure Lactase mit den beiden bei der Deutschen Sammlung von Mikroorganismen und zellkulturen GmbH in Braunschweig hinterlegten Stämmen Lactobacillus delbrueckii LB-VC18 (Hinterlegungsnummer: DSM 23320) und Streptococcus thermophilus ST-VC18 (Hinterlegungsnummer: DSM 23319) kombiniert wird.

Die große Bandbreite der vorstehend genannten Dosierungen erklärt sich dadurch, dass das erfindungsgemäße Mittel bei verschiedensten Schweregraden und auch bei leichteren Lactosestoffwechselstörungen angewendet werden kann. Die Betroffenen reagieren unterschiedlich stark auf eine bestimmte Lactosebelastungen. Außerdem ergeben sich die unterschiedlichen Dosierungen auch dadurch, dass dem Organismus in Abhängigkeit von der jeweiligen Nahrung stark variierende Mengen von Lactose zugeführt werden.

Die nachfolgend erwähnten Kapselgrößen beziehen sich auf die von Capsugel Belgien BVBA, Bornern, Belgien definierten Größen.

Ein erfindungsgemäßes Mittel in Kapseln der Größe 1 könnte pro Kapsel z.B. 70 mg Lactase (Aktivität der Lactase 50.000 FCC/g), 40 mg Streptococcus thermophilus (30 Mrd. cfu/g), 30 mg Lactobacillus bulgaricus (50 Mrd. cfu/g) und 200 mg Dicalciumphosphat enthalten.

Werden Kapseln z.B. der Größe 3 eingesetzt, dann können diese pro Kapsel 70 mg Lactase (Aktivität der Lactase 50.000 FCC/g), 30 mg Streptococcus thermophilus (30 Mrd. cfu/g), 20 mg Lactobacillus bulgaricus (50 Mrd. cfu/g) und 100 mg Dicalciumphosphat enthalten

Ein weiteres Zusammensetzungsbeispiel für die Herstellung von Kapseln besteht aus Kapseln der Größe 2, die 150 mg Lactase (Aktivität der Lactase 50.000 FCC/g), 100 mg Streptococcus thermophilus (30 Mrd. cfu/g), 50 mg Lactobacillus bulgaricus (50 Mrd. cfu/g) und 250 mg Dicalciumphosphat enthalten

Ein weiteres Zusammensetzungsbeispiel für die Herstellung von Kapseln besteht aus Kapsein der Größe 3, die 66 mg Lactase (Aktivität der Lactase 50.000 FCC/g), 10 mg Streptococcus thermophilus (30 Mrd. cfu/g). 6 mg Lactobacillus bulgaricus (50 Mrd. cfu/g) und 130 mg Dicalciumphosphat enthalten.

Das erfindungsgemäße Mittel kann pro Dosiseinheit beispielsweise zwischen 500 und 30.000 FCC-Einheiten Lactase und zwischen 10 Mio, cfu und 200 Mrd. cfu Lactobacillus bulgaricus und/oder zwischen 10 Mio, cfu und 200 Mrd. cfu Streptococcus thermophilus enthalten. Statt dieser beiden Mikroorganismen oder zusätzlich zu einem oder beiden dieser Mikroorganismen kann das erfindungsgemäße Mittel auch jeweils zwischen 10 Mio. cfu und 200 Mrd. cfu der folgenden Milchsäurebakterien enthalten: Lactobacillus delbrueckii (andere subsp. als Lactobacillus bulgaricus), Lactobacillus acidophilus, Lactobacillus rhamnosus, Lactobacillus casei, Lactobacillus casei, Lactobacillus paracasel, Lactobacillus brevis, Lactobacillus plantarum, Lactobacillus salivarius, Lactobacillus rheuteri, Lactococcus lactis, Pediococcos acidilacti, Bifidobacterium lactis, Bifidobacterium bifidum, Bifidobacterium breve, Bifidobacterium longum, Bifidobacterium infantis.
Außerdem können geeignete Zusatzstoffe in erforderlicher Menge eingesetzt werden.

Die Erfindung kann für medizinische Zwecke und nichtmedizinische Zwecke z.B. als Arzneimittel, Medizinprodukt oder Lebensmittel bereitgestellt werden.

Wenn im folgenden der Begriff Therapie verwendet wird, so ist dieser in weitem Sinne zu verstehen. Therapie bezieht sich im Sinne dieser Anmeldung nicht nur auf das Therapieren bzw. Behandeln von Krankheiten, sondern betrifft ganz allgemein die Verwendung des erfindungsgemäßen Mittels bzw. der erfindungsgemäßen Mittel bei Lactoseintoleranz und Lactasemangel beim Menschen oder beim Tier (da bei fast allen Säugetieren nach dem Abstillen die Fähigkeit zur Lactoseverdauung nachläßt). Therapie umfaßt im Sinne dieser Anmeldung demen tsprechend insbesondere das Verwenden des erfindungsgemäßen Mittel bzw. der erfindungsgemäßen Mittel, um das Auftreten von (a) Symptomen jeder Art und jeder Ausprägung, (b) Befindlichkeitsstörungen jeder Art und jeder Ausprägung und (c) jeder Form von gesundheitlichen Beeinträchtigungen und Beschwerden zu verhindern, zu beseitigen, zu vermindern oder diesen vorzubeugen, die infolge der Zufuhr von Lactose oder lactosehaltigen Lebensmittel oder durch Freisetzung von Lactose im Verdauungstrakt eines Menschen oder Tieres aus anderen Stoffe wie z.B. Arzneimittel auftreten können. Therapie umfaßt im Sinne dieser Anmeldung ferner das Beseitigen, das Verringern, das Vorbeugen und das Verhindern von Völlegefühl, Blähungen, Bauchschmerzen, kolikartigen Bauchschmerzen, Koliken, Durchfällen, wässrigen Durchfällen, Darmgeräuschen, Bauchkrämpfen, Aufstoßen, Übelkeit, Erbrechen, Flatulenz oder vermehrter intestimaler Gasproduktion. Ferner umfaßt Therapie im Sinne dieser Erfindung die Verminderung der Bioverfügbarkeit von Lactose im menschlichen oder tierischen Organismus und die Verminderung der für den menschlichen oder tierischen Organismus oder für in selbigen siedelnden Darmbakterien zur Verfügung stehenden Lactosemenge.

## Patentansprüche

1. Eine Zusammensetzung bestehend aus:
(a) einer Lactase; und
(b) einem oder mehreren Lactase-bildenden Mikroorganismen, ausgewählt aus *Lactobacillus bulgaricus, Lactobacillus delbrueckii, Lactobacillus acidophilus, Lactobacillus rhamnosus, Lactobacillus casei, Lactobacillus paracasei, Lactobacillus brevis, Lactobacillus plantarum, Lactobacillus soliverius, Lactobacillus rheuteri, Lactobacillus lactis, Pediococcus acidilacti, Bifidobacterium lactis, Bifidobacterium bifidum, Bifidobacterium breve, Bifidobacterium longum, Bifidobacterium infantis* und *Steptococcus thermophilus;*
aber ausgenommen (i) flüssige Milchprodukte, die die Lactase und *Lactobacillus acidophilus* als alleinigen Lactase-bildenden Mikroorganismus enthalten und (ii) flüssige Milchprodukte, die die Lactase und die drei Mikroorganismen *Lactobacillus bulgaricus, Lactobacillus acidophilus* und *Steptococcus thermophilus* in Kombination enthalten.

2. Eine Zusammensetzung nach Anspruch 1, **dadurch gekennzeichnet, dass** die Lactase keine Lactase ist, die aus einem oder mehreren der besagten Mikroorganismen stammt.

3. Eine Zusammensetzung nach Anspruch 1 oder Anspruch 2, **dadurch gekennzeichnet, dass** die Lactase bei einem pH-Wert von 3 bis 6 aktiv ist.

4. Eine Zusammensetzung nach einem oder mehreren der Ansprüche 1 bis 3, **dadurch gekennzeichnet, dass** die Lactase aus einem Mikroorganismus stammt, und die Zusammensetzung im Wesentlichen von dem Mikroorganismus, von dem die Lactase stammt, frei ist.

5. Eine Zusammensetzung nach einem oder mehreren der Ansprüche 1 bis 4, enthaltend *Lactobacillus bulgaricus* und/oder *Steptococcus thermophilus.*

6. Eine Zusammensetzung nach Anspruch 5, enthaltend *Lactobacillus bulgaricus* und *Steptococcus thermophilus.*

7. Eine Zusammensetzung nach einem oder mehreren der Ansprüche 1 bis 6, **dadurch gekennzeichnet, dass** der Lactase-bildende Mikroorganismus bzw. die Lactase-bildenden Mikroorganismen in getrockneter Form vorliegen.

8. Eine Zusammensetzung nach einem oder mehreren der Ansprüche 1 bis 7, **dadurch gekennzeichnet, dass** die Zusammensetzung in dosierter Form vorliegt.

9. Eine Zusammensetzung nach Anspruch 8, **dadurch gekennzeichnet, dass** es sich bei der dosierten Form um Kapseln, Tabletten oder Dragees handelt.

10. Eine Zusammensetzung nach einem oder mehreren der Ansprüche 8 bis 9, **dadurch gekennzeichnet, dass** die Zusammensetzung zwischen 2000 und 6000 FCC Enzymeinheiten der Lactase enthält, zwischen 100 Millionen und 2 Milliarden koloniebildende Einheiten *Lactobacillus bulgaricus* und zwischen 100 Millionen und 2 Milliarden kolonlebildende Einheiten *Streptococcus thermophilus.*

11. Eine Zusammensetzung nach einem oder mehreren der Ansprüchen 1 bis 10, **dadurch gekennzeichnet, dass** die Zusammensetzung im Wesentlichen frei von Lactose ist.

12. Eine Zusammensetzung nach einem oder mehreren der Ansprüchen 1 bis 11, **dadurch gekennzeichnet, dass** die Zusammensetzung (a) in der Form eines Arzneimittels, (b) in der Form eines Medizinproduktes oder (c) in der Form eines Lebensmittels vorliegt,

13. Eine Zusammensetzung nach einem oder mehreren der Ansprüche 1 bis 12 zur Verwendung in der Medizin.

14. Eine Zusammensetzung nach einem oder mehreren der Ansprüche 1 bis 12 zur Verwendung in der Prophylaxe oder Therapie von Lactoseintoleranz und/oder durch Lactasemangel bedingten Zuständen.

15. Verwendung einer Zusammensetzung nach einem oder mehreren der Ansprüche 1 bis 12 in der Prophylaxe oder Therapie von Lactoseintoleranz und/oder durch Lactasemangel bedingten Zuständen.
